# EUROPEAN PATENT APPLICATION

(11) **EP 3 693 452 A1**
(43) Date of publication of application: **12.08.2020**
(21) Application number: 18864955.2
(22) Date of filing: 02.10.2018
(51) Int. Cl.: C12M 1/00, C12M 3/00, C12N 5/071

(54) **CELL CULTURE CONTAINER, METHOD FOR ACQUIRING CELLS, AND METHOD FOR CULTURING CELLS**

(30) Priority: 03.10.2017 JP 2017193584
(71) Applicant: UNIVERSITY PUBLIC CORPORATION OSAKA, Osaka-shi Osaka 540051 (JP); Tokyo Women's Medical University, Tokyo 162-8666 (JP); Nikon Corporation, Tokyo 108-6290 (JP)
(72) Inventor: KOJIMA Chie, Osaka 5998531 (JP); SHIMIZU Tatsuya, Tokyo 162-8666 (JP); HARAGUCHI Yuji, Tokyo 162-8666 (JP); KAWANO Takeshi, Tokyo 108-6290 (JP); TAKI Yusuke, Tokyo 108-6290 (JP); YOKOYAMA Kaede, Tokyo 108-6290 (JP)
(74) Representative: Hoffmann Eitle
(86) International application number: PCT/JP2018/036897
(87) International publication number: WO 2019/069931

(57) **Abstract**

A cell culture container includes: an inlet (21) through which a fluid is supplied; an outlet (22) through which a fluid is discharged; and a flow path (23) configured to connect the inlet to the outlet and accommodate a cell culture substrate (14) obtained by dispersing gold nanoparticles (13) in a gel capable of being denatured by heating. A method for acquiring cells includes: a step of selecting cells to be acquired from cells cultured in the cell culture container; a step of irradiating the gel in the vicinity of the selected cells with light; a step of causing a liquid to flow through the flow path of the cell culture container; and a step of recovering cells from the outlet of the cell culture container.

## Description

### TECHNICAL FIELD

The present invention relates to a cell culture container, a method for acquiring cells, and a method for culturing cells.

Priority is claimed on Japanese Patent Application No. 2017-193584, filed October 3, 2017, the content of which is incorporated herein by reference.

### BACKGROUND ART

In order to recover cells cultured using a culture substrate more efficiently, for example, there has been proposed a method for culturing cells above a culture substrate including a thermosensitive polymer and recovering the cells using a change in temperature (refer to Patent Document 1).

### Citation List

### Patent Literature

[Patent Document 1]
Japanese Unexamined Patent Application, First Publication No. H11-349643

### DISCLOSURE OF INVENTION

### Technical Problem

According to a first aspect of the present invention, a cell culture container includes: an inlet through which a fluid is supplied; an outlet through which a fluid is discharged; and a flow path configured to connect the inlet to the outlet and accommodate a cell culture substrate obtained by dispersing gold nanoparticles in a gel capable of being denatured by heating.

According to a second aspect of the present invention, a method for acquiring cells includes: a step of selecting cells to be obtained from cells cultured in the cell culture container according to the first aspect; a step of irradiating the gel in the vicinity of the selected cells with light; a step of causing a liquid to flow through the flow path of the cell culture container; and a step of recovering cells through the outlet of the cell culture container.

According to a third aspect of the present invention, a method for culturing cells includes: culturing cells acquired through the method for acquiring cells according to the third aspect.

### BRIEF DESCRIPTION OF DRAWINGS

Fig. 1 is a cross-sectional view of a cell culture container in an embodiment.
Fig. 2 is a conceptual diagram illustrating a constitution of a cell acquisition system including the cell culture container in the embodiment.
Fig. 3 is a conceptual diagram illustrating the constitution of the cell culture container in the embodiment.
Fig. 4 is a diagram schematically illustrating an operation after cells are cultured and before target cells are detached using the cell culture container in the embodiment.
Fig. 5 is a diagram schematically illustrating an operation of irradiating target cells with a laser beam using the cell culture container in the embodiment.
Fig. 6 is a diagram schematically illustrating an operation after target cells are irradiated with a laser beam using the cell culture container in the embodiment.
Fig. 7 is a flowchart describing a flow of a method for producing a cell culture container in the embodiment.
Fig. 8 is a flowchart describing a flow of a method for acquiring cells using a cell culture container in an embodiment.
Fig. 9 is a diagram illustrating a top view and a cross-sectional view of a cell culture container in an example.

### BEST MODE FOR CARRYING OUT THE INVENTION

### (First embodiment)

A cell culture container, a method for acquiring cells, and the like in an embodiment will be described below with reference to the drawings as appropriate.

It is necessary to efficiently detach and recover specific cells such as cells induced to differentiate from induced pluripotent stem cells (iPS cells) among cells cultured above a cell culture substrate in many cases.

The present inventors found that it is possible to efficiently recover only desired cells by disposing a cell culture substrate in a flow path, causing a local change in temperature to occur in the cell culture substrate using light from the outside to weaken the adhesion of the desired cells to the cell culture substrate, causing a liquid to flow through the flow path to detach the cells using a shear stress of the liquid, and recovering the cells together with the liquid.

A cell culture container in this embodiment includes an inlet through which a fluid is supplied and an outlet through which a fluid is discharged. In addition, it is possible to collectively recover cells detached using a fluid flowing through the cell culture container by accommodating the cell culture substrate in a flow path configured to connect the inlet to the outlet.

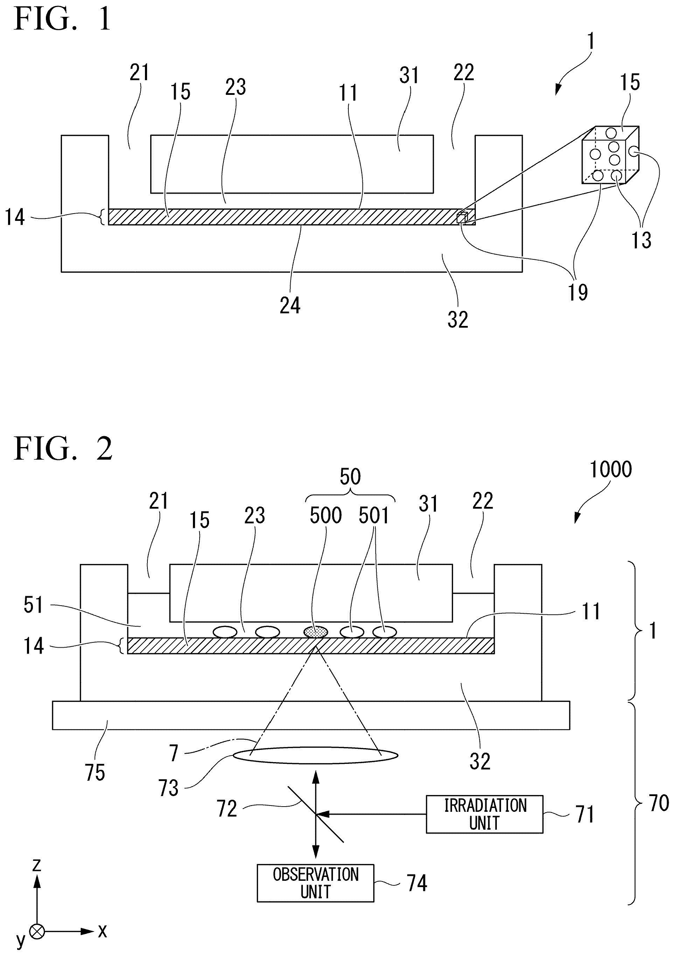

Fig. 1 is a cross-sectional view of a cell culture container 1 in this embodiment. The cell culture container 1 includes an inlet 21 through which a fluid is supplied, an outlet 22 through which a fluid is discharged, and a flow path 23 to connect the inlet to the outlet. A cell culture substrate 14 obtained by dispersing gold nanoparticles in a gel 15 capable of being denatured by heating is accommodated in the flow path 23. Gold nanoparticles 13 have been illustrated by schematically enlarging a micro volume element 19 inside the gel 15.

A fluid refers to a liquid or a gas. As the liquid, for example, various culture solutions and buffer solutions are selected and used in accordance with the type of cells. In addition, as the gas, for example, air is used.

The gold nanoparticles 13 can be particles having sizes with excellent photothermal conversion characteristics, and particularly, may have sizes in which surface plasmon resonance absorption (SPR) occurs. A volume-average diameter of the gold nanoparticles 13 measured using a laser diffraction type particle size distribution analyzer can be, for example, 1 nm or more and less than 200 nm, 10 nm or more and less than 100 nm, 30 nm or more and less than 70 nm. A concentration of the gold nanoparticles 13 in the gel 15 may be, for example, 100 µM or more and less than 1000 µM and 250 µM or more and 500 µM or less. If the concentration of the gold nanoparticles 13 is low, a calorific value of the gold nanoparticles 13 is low. Thus, thermal denaturation of the cell culture substrate does not easily occur and a cell detachment success probability tends to decrease. On the other hand, if the concentration of the gold nanoparticles 13 is high, a local rise in temperature increases. Thus, it is difficult to control a temperature and cytotoxicity tends to increase.

The gold nanoparticles 13 may be further stabilized in a gel using a protective agent, for example, by being encapsulated in a dendrimer or modified with molecules having an affinity for a gel.

The gel 15 denatures when a temperature thereof rises from room temperature to a predetermined temperature. In this embodiment, the term "denaturation" means that the gel 15 undergoes a structural change which causes easy detachment of cells due to a rise in temperature. When the gel 15 is a collagen gel or a gelatin gel, the term "denaturation" includes, for example, solation, aggregation, decomposition into small molecules, and the like which are states caused due to changes in secondary or tertiary structures of collagen proteins. A temperature at which the gel 15 denatures is, for example, 60 °C or lower, 50 °C or lower, and 40 °C or lower. Although a material of the gel 15 is not particularly limited as long as it enables cells to be obtained through detachment which will be described later, for example, the material of the gel 15 may be a collagen gel or a gelatin gel. It is also possible to use a gel obtained by mixing two or more kinds of polymers.

At least one of a bottom portion 32 and an upper portion 31 of the cell culture container 1 is formed of a light-transmitting material. The cell culture container 1 can be constituted in any shape as long as the technical features of the cell culture container 1 in this embodiment are not impaired.

Materials of the bottom portion 32 and the upper portion 31 of the cell culture container 1 may have oxygen permeability. For example, an oxygen-permeable polymer resin and the like such as an acrylic resin or a polystyrene resin may be used. When the materials have oxygen permeability, it is possible to supply oxygen required for culturing a cell 50 from the outside.

Fig. 2 is a conceptual diagram illustrating a cell recovery system 1000 configured to acquire cells using the cell culture container. The cell acquisition system 1000 includes the cell culture container 1 and an inverted microscope 70.

In the cell culture container 1, the cells 50 are cultured in a culture solution 51 above a mounting surface 11 of the cell culture substrate 14.

Cells to be isolated and acquired among the cells 50 which are being cultured in the cell culture substrate 14 are a selected cell 500 and other cells are non-selected cells 501. Although a form of the selected cell 500 is not particularly limited, for example, a cell in which a gene and the like has been appropriately modified and characteristics have been revealed using a discriminating means such as a reporter gene, a cell in which initialization, appropriate differentiation, and the like are induced and which has characteristics capable of being discriminated structurally, and the like may be used.

The selected cell 500 may be a single cell 50 as described above or may be a plurality of cells 50 or a colony constituted to include a plurality of cells 50.

The inverted microscope 70 includes an irradiation unit 71, a dichroic mirror 72, a lens system 73, an observation unit 74, and a support base 75.

The cell recovery system 1000 may be constructed using an upright microscope.

The irradiation unit 71 emits a laser beam. A wavelength of the laser beam emitted from the irradiation unit 71 is set in a wavelength range in which the gold nanoparticles 13 exhibit photothermal conversion characteristics. Particularly, the wavelength may be set in a wavelength range in which surface plasmon resonance absorption (SPR) occurs. A laser beam radiated to the gel 15 may have a wavelength and energy in which significant damage is not caused when radiated to cells. The wavelength of the laser beam emitted from the irradiation unit 71 is set to, for example, 400 nm or more and less than 1200 nm, 450 nm or more and less than 900 nm, 532 nm, and the like. An output of a laser beam incident on the cell culture container 1 can be, for example, 0.1 mW or more and less than 1000 mW and 0.4 mW or more and less than 100 mW. A wavelength and energy of a laser beam are appropriately adjusted so that a temperature of the gel 15 efficiently increases and the gel 15 efficiently denatures without damaging cells. Light emitted from the irradiation unit 71 is incident on the dichroic mirror 72.

As long as the vicinity of the selected cell 500 can be selectively irradiated with light, the light emitted from the irradiation unit 71 is not particularly limited to a laser beam and may be non-coherent monochromatic light or light composed of light in a certain wavelength range.

The dichroic mirror 72 reflects the laser beam from the irradiation unit 71 and visible light from the cell culture container 1 is transmitted through the dichroic mirror 72 and emitted to the observation unit 74. A light direction of the laser beam reflected by the dichroic mirror 72 is adjusted so that an appropriate position is irradiated with the laser beam using a galvanomirror (not shown) or the like and the laser beam is transmitted through the lens system 73 and incident on the cell culture container 1. The laser beam incident on the cell culture container 1 is transmitted through the bottom portion 32 and the cell culture substrate 14 of the cell culture container 1 and then converges at a predetermined position in the vicinity of the selected cell 500 in the cell culture substrate 14. In Fig. 2, the converging laser beam 7 is schematically illustrated using an alternating long and short dash line.

A constitution of an irradiation optical system of the laser beam 7 is not particularly limited as long as the laser beam 7 can converge to a predetermined position.

Furthermore, a constitution in which an irradiation position of a laser beam is fixed and the support base 75 is moved so that the laser beam converges in the vicinity of the selected cell 500 may be provided.

A convergence position of the laser beam 7 in the cell culture substrate 14 can be, for example, a position as close as possible below the selected cell 500 without damaging the selected cell 500 and is determined on the basis of a spot diameter of the laser beam 7, a point spread function (PSF), and parameters based on the PSF. The convergence position of the laser beam 7 in the cell culture substrate 14 may be, for example, within a range in which the convergence position is immediately below the selected cell 500 and a depth thereof is less than 100 µm or within a range in which the convergence position is less than a radius of 100 µm from the selected cell 500. Furthermore, if there is no problem in cytotoxicity, the selected cell 500 may be set as a convergence position.

If the gold nanoparticles 13 photothermally convert the laser beam 7 from the irradiation unit 71 and a gel in the vicinity of the convergence position of the laser beam 7 denatures, the physical or chemical properties of a gel serving as a scaffold of the selected cell 500 or the surrounding environment change. Thus, the adhesion of the selected cell 500 to the mounting surface 11 is weakened. Therefore, it is possible to detach and take out only the selected cell 500 from the gel by causing a liquid to flow through the flow path 15. To be specific, for example, it is possible to cause a liquid to flow through the flow path 15 by directly or indirectly connecting a liquid storage tank to the inlet 21 using a tube or the like and sending the liquid through the inlet 21 using a pump or by suctioning the liquid through the outlet 22. Since the selected cell 500 detached from the gel using the shear stress of the liquid is caused to flow through the outlet 22 while pressed by the liquid, it is possible to recover the selected cell 500 together with the liquid through the outlet 22.

The observation unit 74 includes an eyepiece or the like and enables a user to observe visible light from the cell culture container 1 lit by a lighting (not shown). The user can see the visible light from the cell culture container 1 and appropriately position the cell culture container 1 by moving the support base 75.

A constitution in which an image of the cell culture container 1 is acquired by performing laser scanning using a laser beam source and a galvanomirror different from those of the irradiation optical system of the laser beam 7 and displayed on a display device (not shown) may be used.

The support base 75 supports the cell culture container 1. The support base 75 can move in X, Y, and Z directions using a moving mechanism (not shown) and thus the cell culture container 1 can be adjusted to have an arbitrary position. The support base 75 is constituted to include, for example, glass having a transparent heating element formed thereon, the laser beam 7 is transmitted to the bottom portion 32 of the cell culture container 1, and a temperature of the entire cell culture container 1 is controlled.

It is also possible to use the support base 75 having an opening portion formed in a portion serving as an optical path of the laser beam 7 in accordance with a size, a structure, and the like of the cell culture container 1.

The cell culture substrate 14 in this embodiment may be formed by laminating a plurality of gels having gold nanoparticles at different concentrations. For example, the cell culture substrate 14 can have a layer formed of a gel which does not include gold nanoparticles or includes gold nanoparticles with a concentration lower than that of the gel 15 disposed below the gel 15 including gold nanoparticles. A thickness of the gel 15 which is a heat-generating layer is made substantially uniform on a surface by providing the layer formed of the plurality of gels. Thus, it is easy to adjust irradiation conditions of the laser beam 7 and it is easy to isolate cells even when an irradiation site is changed.

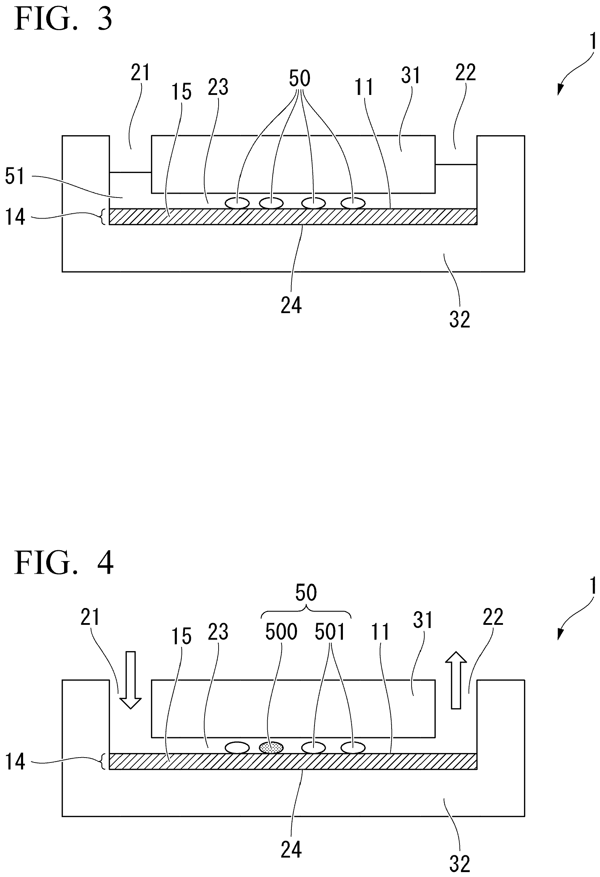

Fig. 3 illustrates a cross-sectional view of an XZ plane of the cell culture container 1.

In the cell culture substrate 14 in this embodiment, the thickness of the gel 15 can be, for example, 0.4 mm or more, 0.5 mm or more and 1.7 mm or less, 0.5 mm or more and 1.2 mm or less. If the thickness of the gel 15 is too thin, it tends to be difficult to make the gel 15 uniform or flat. On the other hand, if the thickness of the gel 15 is too thick, an amount of culture solution filling the flow path 23 decreases and cell culture tends to be difficult. Furthermore, since light reaching the gel 15 may be attenuated and a calorific value of the gold nanoparticles 13 in the vicinity of the cell 50 may decrease in some cases, the acquisition of the cell 50 is likely to be difficult.

Here, the thickness of the gel 15 refers to an average thickness from a surface of the bottom portion 32 of the cell culture container 1.

After the cell culture, the gel 15 may be heated before irradiation with the laser beam 7. Thus, it is possible to shorten a time over which a laser beam is radiated to denature a gel and it is possible to reduce an influence of a laser beam on the selected cell 500. As an example of a method for heating the gel 15, as illustrated in Fig. 4, a method for supplying a liquid (for example, a culture solution) at a temperature lower than a temperature at which a gel totally denatures, for example, at a temperature of 37 °C or higher and less than 40 °C from the inlet 21 to the flow path 23 in a direction of an arrow may be exemplified. By supplying a liquid 51 from the inlet 21 to the flow path 23 at a temperature lower than a temperature at which the gel totally denatures, a temperature around the selected cell 500 increases.

In order to increase a temperature of the gel 15, a heating element of the support base 75 may be used, the cell culture container 1 may be placed in a temperature-controllable incubator, or a combination of two or more of these methods may be used.

Also, the liquid 51 in the flow path 23 tends to minimize a rise in gel surface temperature in the vicinity of the selected cell 500 when irradiated with the laser beam 7 and tends to reduce the efficiency of detachment of the selected cells. For this reason, before the irradiation with the laser beam 7, air may be supplied into the inlet 21 or the liquid 51 may be removed through the outlet 22 by suctioning the liquid through the outlet 22. Although the removal of the liquid 51 may not be completed and some of the liquid may remain in the flow path 23, if an amount of the remaining liquid is too large, a rise in gel surface temperature in the vicinity of the selected cell 500 is suppressed. Thus, it is difficult to increase the efficiency of acquiring the selected cell 500. Furthermore, if too much of the liquid is removed, the cell 50 is likely to be damaged. For example, the liquid 51 may be removed to an extent that a liquid is in a range of 0.1 mm to 0.8 mm from a mounting surface 11 of a gel 14.

Here, if the liquid is removed, when a liquid flows through the flow path 23 after irradiation with a laser, since an interface between a gas and the liquid passes through the mounting surface 11 of the gel 14, a larger shear stress is applied to the selected cell 500 and the effect of easy detachment is also obtained.

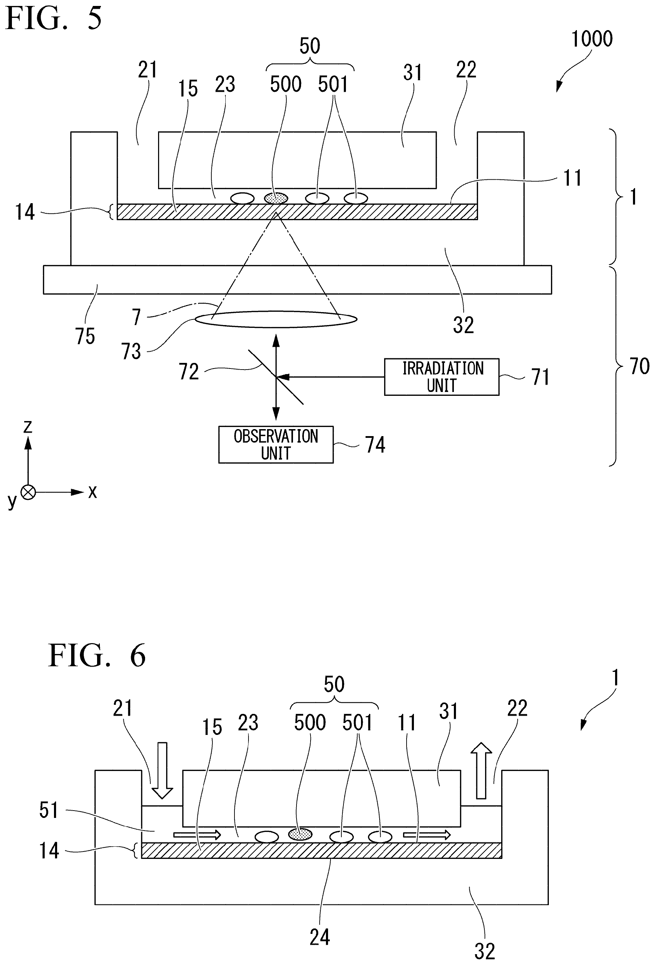

Fig. 5 is a diagram schematically illustrating an operation of irradiating a target cell with the laser beam 7. When the liquid 51 is removed, if the vicinity of the selected cell 500 is then irradiated with the laser beam 7, the gold nanoparticles 13 in the gel 15 undergo photothermal conversion and the physical or chemical properties of a gel serving as a scaffold of the selected cell 500 or a surrounding environment change. Thus, a force adhering the selected cell 500 to the mounting surface 11 is weakened. In addition, if a liquid flows through the flow path, the selected cell 500 is detached from the mounting surface 11 due to a shear stress.

Fig. 6 is a diagram schematically illustrating an operation after a target cell is irradiated with the laser beam 7. Since the selected cell 500 whose adhesiveness to the mounting surface 11 is weakened due to the laser beam 7 is detached from the mounting surface 11 due to a shear stress of a liquid by causing a liquid to flow through the flow path 15 and is caused to flow through the outlet 22 while pressed by the liquid, it is possible to recover the selected cell 500 together with the liquid through the outlet 22.

A speed at which the liquid flows through the flow path 15 can be appropriately determined by a person of ordinary skill in the art.

### (Method for producing cell culture substrate 1)

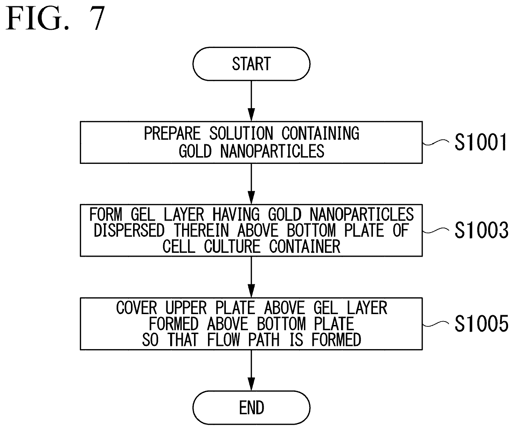

Fig. 7 is a flowchart describing a flow of a method for producing the cell culture container 1 in this embodiment.

In Step S1001, a solution containing the gold nanoparticles 13 having a predetermined volume-average diameter is prepared. For example, as in the method described in Japanese Unexamined Patent Application, First Publication No. 2013-233101, a seed nucleus made of gold can be grown and obtained in a solution containing gold ions (such as HAuCl4) in the presence of a reducing agent (such as ascorbic acid, hydroquinone, or citric acid). If Step S1001 has ended, the process proceeds to the process of Step S1003.

Regarding the volume-average diameter of the gold nanoparticles 13, the adjusted gold nanoparticles 13 can be measured using a laser diffraction type particle size distribution analyzer. In the case of simply performing a measurement without using the particle size distribution analyzer, it is possible to perform imaging using a transmission electron microscope and perform a measurement and a calculation using analysis software or the like.

In Step S1003, a mixed solution containing collagen and the gold nanoparticles 13 is prepared using the solution containing the gold nanoparticles 13 prepared in Step S1001 and allowed to stand while applied above the bottom plate 32 of the cell culture container 1. If the mixed solution is solidified, the gold nanoparticles 13 are dispersed and embedded in a collagen gel. If Step S1003 has ended, the process proceeds to the process of Step 1005.

In Step S1005, the upper plate 31 is placed above a gel layer formed above the bottom plate 32 of the cell culture container 1 so that the flow path 23 is formed. Although the height of the flow path 23 can vary depending on a thickness of the gel layer, for example, the height thereof can be, for example, 0.2 mm or more and 2.0 mm or less, 0.5 or more and 1.5 mm, and 0.8 mm or more and 1.2 mm or less from a gel surface. If Step S1005 has ended, the process ends.

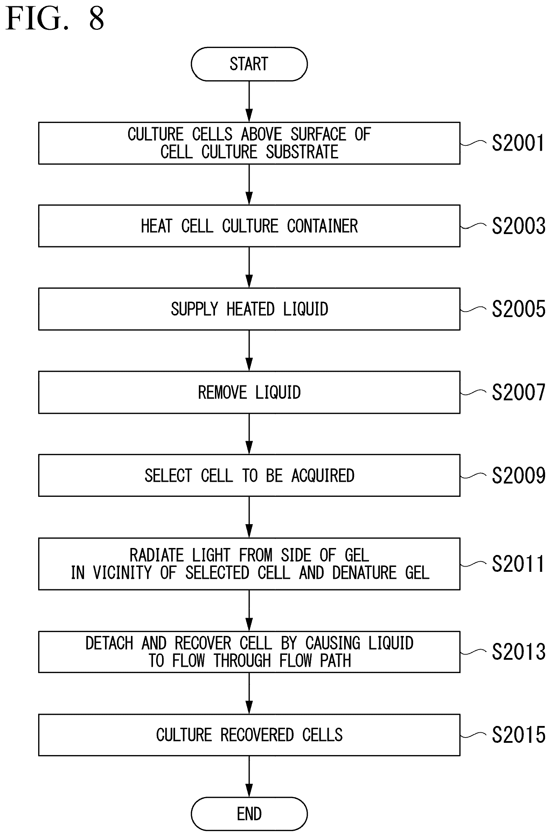

Fig. 8 is a flowchart describing a flow of a method for acquiring cells using the cell culture container 1 and a method for producing cells in this embodiment. In Step S2001, cells are cultured above a surface of the cell culture substrate 14, that is, the mounting surface 11. If Step S2001 has ended, the process proceeds to the process of Step S2003.

In Step S2003, a temperature of the support base 75 is appropriately adjusted and the cell culture container 1 is heated to a temperature less than a temperature at which total denaturation of the gel 15 occurs. By increasing a temperature of the cell culture container 1 before the irradiation with the laser beam 7, it is possible to shorten a time of irradiation with the laser beam 7 and it is possible to reduce cytotoxicity to the selected cell 500. If Step S2003 has ended, the process may proceed to the process of Step S2005 and then Step S2003 may be continuously performed from Step S2005 to any of Step S2005 to S2013.

In Step S2005, the liquid (for example, the culture solution) 51 heated to a temperature less than the temperature at which total denaturation of the gel 15 occurs is supplied. By increasing the temperature of the liquid 51 before the irradiation with the laser beam 7, it is possible to shorten a time of irradiation with the laser beam 7 and it is possible to reduce an influence on the selected cell 500. If Step 2005 has ended, the process proceeds to Step 2007. The order of Step S2003 and Step S2005 may be changed as appropriate or operations thereof may be performed in parallel with each other.

In Step S2007, the liquid 51 in the cell culture container 1 is removed from the flow path 23. The removal of the liquid 51 is performed by supplying air through the inlet 21 of the cell culture container 1 or by suctioning the liquid 51 through the outlet 22. If Step S2007 has ended, the process proceeds to the process of Step S2009. In Step S2009, the cells 50 (the selected cells 500) to be acquired are selected from the cells 50 cultured above the mounting surface 11. If necessary, a cell 50 in which a fluorescent protein is expressed or a cell 50 having structural characteristics is selected.

The order from Step S2005 to Step S2009 may be changed. For example, the liquid 51 may be removed after the heated liquid 51 is supplied and the cell 50 is selected or the heated liquid 51 may be supplied after the cell 50 is selected and the liquid 51 may be removed.

In Step S2011, a convergence position in the vicinity of the selected cell 500 in the gel 15 is irradiated with the laser beam 7. By radiating the laser beam 7 from a surface side opposite to the mounting surface 11 of the gel 15, it is possible to prevent direct light from coming into contact with the cell 50 and causing cell damage. The denaturation of the gel weakens the binding between the selected cell 500 and the mounting surface 11. If Step S2011 has ended, the process proceeds to the process of Step S2013. When there are a plurality of selected cells 500 to be collected together, Step S2009 and Step S2011 are repeatedly performed a plurality of times and if Step S2011 has been performed on all of the selected cells 500, the process proceeds to the process of Step S2013. Furthermore, when Step S2009 for selecting cells is performed before Step S2005 for supplying the heated culture solution and Step S2007 for removing the culture solution and when there are a plurality of selected cells 500, Step S2009 may be repeatedly performed a plurality of times to select all cells and then Step S2005 and Step S2007 may be performed. Subsequently, Step 2011 may be repeatedly performed and all of the selected cells 500 may be irradiated with a laser.

In Step S2013, by causing a liquid to flow through the flow path 23 of the cell culture container 1, the selected cell 500 is detached from the mounting surface 11 of the gel 15 due to a shear stress of the liquid and flow to the outlet 22. Thus, the selected cells 500 are recovered through the outlet 22. In Step 2015, the recovered cells 500 can be placed on another medium or the like and cultured. If Step S2015 has ended, the process ends. In Step S2013, if the selected cells 500 are recovered, the process may return to the process of Step S2003 and other cells 50 may be acquired as the selected cell 500. Furthermore, the recovered cells may be directly used for various purposes such as in clinical use, research, and industrial uses.

According to the above-described embodiment, the following action and effects can be obtained.
(1) The cell culture container 1 in this embodiment includes the inlet through which the fluid is supplied, the outlet through which the fluid is discharged, and the flow path configured to connect the inlet to the outlet and accommodate the cell culture substrate obtained by dispersing the gold nanoparticles in the gel capable of being denatured by heating. Thus, by causing the liquid to flow through the flow path, it is possible to collectively recover only the selected cell and it is possible to increase the number of cells to be recovered per unit time.
(2) The method for acquiring cells in this embodiment includes a step of selecting cells to be acquired from cells cultured in the cell culture container of the first embodiment, a step of irradiating the gel in the vicinity of the selected cells with light, a step of causing the liquid to flow through the flow path of the cell culture container, and a step of recovering the cells through the outlet of the cell culture container. Thus, it is possible to recover the selected cells through a running flow generated in the flow path and it is possible to increase the number of cells to be recovered per unit time.

The present invention is not limited to the contents of the above embodiment. Other embodiments which can be conceivable within the scope of the technical concept of the present invention are also included in the scope of the present invention.

### (Example)

Cells of this embodiment were obtained using cardiomyocytes induced to differentiate from iPS cells using the iPS cells.

### (Reagent preparation)

### • Calcium/magnesium-free phosphate-buffered saline [PBS(-)]

NaCl (4.003 g), KCl (0.1003 g), KH₂PO₄ (0.1006 g), and NaH₂PO₄ (0.575 g) were dissolved in deionized water (500 mL) and then sterilized in an autoclave to prepare PBS(-).

### • 10 times inorganic salt medium

Anhydrous CaCh (20.2 mg), MgCl·6H₂O (23.5 mg), KCl (40.4 mg), NaCl (639.7 mg), and NaH₂PO₄·2H₂O (14.1 mg) were added to and dissolved in deionized water (10 mL) to prepare a 10 times inorganic salt medium.

### • Reconstitution solution

NaHCO₃ (219.7 mg) and HEPES (477.12 mg) were added to and dissolved in NaOH (0.05 M; 10 mL) to prepare a reconstitution solution.

### • Diluted hydrochloric acid

A diluted hydrochloric acid (pH 3.0) was prepared by adjusting 0.05 M HCl aqueous solution to pH 3.0 using a pH meter (pH/CONDMETER D-54 manufactured by HORIBA, Ltd.). Here, each of the 10 times inorganic salt medium, the reconstitution solution, and the diluted hydrochloric acid (pH 3.0) was filter-sterilized using a filter (manufactured by ADVANTEC CO., LTD.; pore size of 0.20 µm).

### • Concentrated gold nanoparticle (AuNP) solution

4 mL (2 mL×2) of a growth solution (refer to S. Yagi, et al, JElectrochem Soc, 159, H668 (2012)) which was a solution having grown gold nanoparticles was input into a centrifuge tube and centrifuged at 25 °C at 3000 rpm for 20 minutes. A supernatant solution (1.8 mL×2) was removed, ultrapure water (1.8 mL×2) was added, and the mixture was centrifuged again under the same conditions. Subsequently, the supernatant solution (1.8 mL×2) was removed, the ultrapure water (0.2 mL×2) was added, and a concentrated AuNP solution (Au 500 µM) in which a concentration of a reducing agent was diluted was prepared. A volume-average diameter of the gold nanoparticles was calculated by capturing the adjusted gold nanoparticles using a transmission electron microscope (JEM-2000F manufactured by JEOL Ltd.; accelerating voltage of 200 kV) and performing measurement and calculation using analysis software (Photomeasure (registered trademark) manufactured by KENIS Ltd.).

### (Gel preparation)

On a clean bench (S-1001PRV manufactured by SHOWAKAGAKU CO., LTD.), a collagen gel solution (2.1 mL) was prepared by adding diluted hydrochloric acid (0.42 mL), a concentrated gold nanoparticle (AuNP) solution (0.70 mL), a 10 times inorganic salt medium (0.20 mL), and a reconstitution solution (0.20 mL) to a cell culture substrate (Cellmatrix (registered trademark) I-A manufactured by Nitta Gelatin Inc.; concentration of collagen of 0.3 wt%; pH 3.0; 0.56 mL) in this order under ice cooling. A collagen gel having gold nanoparticles embedded therein was prepared by inputting this mixture into a 96-well plate (manufactured by Nunc) under ice cooling and then incubating this mixture in a direct heat CO₂ incubator at 37 °C for 30 minutes. Subsequently, the gel was washed by adding 100 µL/well of PBS(-) above the gel at 37 °C and incubating it for 6 hours.

### (Preparation of culture chip)

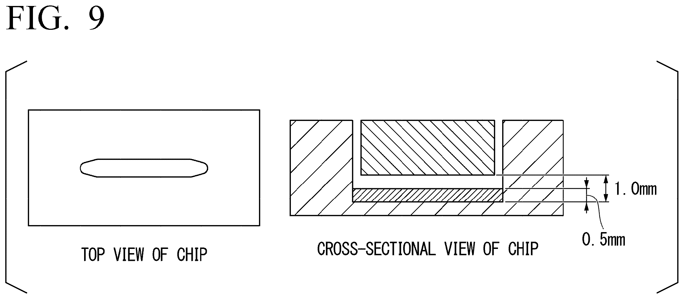

An acrylic plate was bonded to an acrylic plate using a polydimethylsiloxane (PDMS; thickness of 100 µm) sheet so that a flow path having a height of 1.0 mm was formed using a laser beam machine. A collagen gel having gold nanoparticles embedded therein and prepared as described above was added to the flow path until the height of the gel was 0.5 mm and incubated in the direct heat CO₂ incubator at 37 °C for 30 minutes. Subsequently, a culture chip was prepared by bonding an upper plate of the acrylic plate processed so that the height from a gel surface to a bottom surface of the upper plate was 0.5 mm using the PDMS sheet. Fig. 9 illustrates a top view and a cross-sectional view of the prepared culture chip.

### (Cell seeding and detachment)

According to the literature (Biochem Biophys Res Commun, 425; 321:2012), human iPS cells were differentiated into cardiomyocytes in a suspension culture system using a bioreactor.

In the suspension culture system, the human iPS cells adhere to each other without adhering to an inner surface of the bioreactor and proliferate while forming spherical cell aggregates (embryoid bodies). In the embryoid bodies, the above-described cardiomyocyte differentiation proceeded and embryoid bodies containing self-sustaining cardiomyocytes appeared. Cardiomyocyte differentiation was performed for 17 days using this suspension culture bioreactor. Treatment for enzyme or the like was performed to disperse embryoid bodies 17 days after the differentiation and obtain single cells. Single cells were obtained using a mild treatment method (treatment using trypsin/EDTA at a concentration one-fifth of that used for normal cell passage) to prevent cardiomyocyte damage. It was possible to obtain single cells with good reproducibility every time using this method.

The cardiomyocytes (6000 cell/well) dispersed in a normal Dulbecco's modified Eagle's medium for animal cells (DMEM) (containing a 10% fetal calf serum and a 1% penicillin-streptomycin) was caused to flow into the flow path through the inlet to the prepared culture chip and cultured at 37 °C for 4 days.

After completion of the culture, the culture chip was transferred to a thermal insulating chamber (Compact chamber for cell culture C-150AW; manufactured byoo) maintained at a temperature of 37 °C. Subsequently, after tubes were installed in the inlet and the outlet of the culture chip, a culture solution heated to 40 °C was supplyed using a syringe. Subsequently, air was supplyed to remove the culture solution through the outlet. Subsequently, an air displacement cell detachment chip was obtained by installing the thermal insulating chamber on the support base of the microscope, detecting beating myocardium under the microscope, performing setting so that a spot diameter to the cardiomyocytes was the minimum, and radiating light.

As a control, an air-displacement-free detachment chip was obtained in the same manner as described above, except that light was radiated without leaving the culture solution without supplying air.

A photostimulator (a Ti-LAPP FRAP module) was attached to a motorized Ti-E microscope. A constitution in which a laser was introduced into the photostimulator using a fiber and driving could be performed independently of an observation optical system in the XYZ axis direction was provided. Furthermore, for the purpose of performing precise temperature control, the microscope was covered with an insulation box and a double heat insulation structure in which the periphery of the chip was covered with a small incubator (C150-HA manufactured by BLAST) was provided. The culture solution was introduced into the chip after the culture solution input into a 50 mL centrifuge tube was sent to a block heater through a tube using a diaphragm pump and preheated. This is because, when preheating is not performed, a temperature of the culture solution decreases at the time of cell detachment and a collagen gel having gold nanoparticles embedded therein and thermally denatured through laser irradiation might return to a state in which the collagen gel was not thermally denatured in some cases. After the block heater, the tube was connected to a flow meter/a thermometer (SLI-2000 manufactured by Sensirion AG Switzerland) and a flow rate and a temperature of the culture solution were monitored. The tube was connected to a chip after the flow meter/thermometer and to a recovery/waste container after the chip. Air supply was performed by inserting a needle into the tube between the chip and the flow meter/thermometer and inputting air through the needle using a syringe. The observation was performed using a 4 times and 10 times objective lens (Plan-Fluor manufactured by Nikon Corporation).

Light irradiation was performed five times for 2.0 seconds at 1.0 second intervals.

A procedure for cell detachment was as follows. First, target cardiomyocytes were visually detected through 10 times phase contrast observation. Subsequently, about 50 to 100 µL of air was supplied (to an extent that the flow path was filled with air) and the culture solution was removed from the flow path. Moreover, the filter was changed for laser irradiation and the laser was radiated for about 2 to 20 seconds. An irradiation time was adjusted depending on laser power. The culture solution was caused to flow through the flow path by performing suctioning through the outlet or the culture solution was sent through the inlet after the laser irradiation and detachment was performed by applying an external force to cardiomyocytes.

### (Cell recovery)

After the light irradiation, the culture solution kept at 40 °C was supplied at a flow rate of 5 ml/min through the inlet of the culture chip using a syringe and cells detached from the tube installed in the outlet were recovered.

### (Results)

The cardiomyocytes obtained from the recovered culture solution had pulsation. In addition, according to the method of the present invention, it was confirmed that the cells could be recovered without causing any damage to the cells.

When the culture solution was removed before the light irradiation was performed, it was found that target cells were highly likely to be able to be detached/recovered compared to a case in which the cells were not removed.

As a result, it was found that the efficiency of obtaining the selected cells can be increased by removing the culture solution by replacing the air before the light irradiation.

### [Reference Signs List]

1 Cell culture container
7 Laser beam
10 First layer
11 Mounting surface
13 Gold nanoparticle
14 Cell culture substrate
15 Gel
21 Inlet
22 Outlet
24 Flow path
50 Cell
51 Culture solution
70 Microscope
500 Selected cell
1000Cell acquisition system

## Claims

1. A cell culture container, comprising:
an inlet through which a fluid is supplied;
an outlet through which a fluid is discharged; and
a flow path configured to connect the inlet to the outlet and accommodate a cell culture substrate obtained by dispersing gold nanoparticles in a gel capable of being denatured by heating.

2. The cell culture container according to Claim 1, wherein the gel is a collagen gel or a gelatin gel.

3. The cell culture container according to Claim 1 or 2, wherein the height of the flow path is 0.2 mm or more and 2.0 mm or less from a surface of the gel.

4. A method for acquiring cells, comprising:
a step of selecting cells to be obtained from cells cultured in the cell culture container according to any one of Claims 1 to 3;
a step of irradiating the gel in the vicinity of the selected cells with light;
a step of causing a liquid to flow through the flow path of the cell culture container; and
a step of recovering cells through the outlet of the cell culture container.

5. The method for acquiring cells according to Claim 4, wherein, before the step of irradiating the gel with light, a step of supplying a liquid having a temperature of 37 °C or higher and less than 40 °C to the flow path of the cell culture container is provided.

6. The method for acquiring cells according to Claim 4 or 5, wherein, immediately before the step of irradiating the gel with light, a method for removing all or a part of a liquid from the flow path is provided.

7. The method for acquiring cells according to Claim 6, wherein the step of removing the liquid is a step of supplying a gas through the inlet of the cell culture container or a step of suctioning a liquid through the outlet of the cell culture container.

8. A method for culturing cells, comprising:
culturing cells acquired through the method for acquiring cells according to any one of Claims 4 to 7.
